# EUROPÄISCHE PATENTSCHRIFT

(11) **EP 3 710 830 B1**
(45) Veröffentlichungstag und Bekanntmachung des Hinweises auf die Patenterteilung: **17.07.2024**
(21) Anmeldenummer: 18826714.0
(22) Anmeldetag: 21.12.2018
(51) Int. Cl.: G01N 33/28, B03C 1/28

(54) **SENSORVORRICHTUNG FÜR METALLPARTIKEL IN FLUIDEN**
SENSOR APPARATUS FOR METAL PARTICLES IN FLUIDS
DISPOSITIF DE DÉTECTION DE PARTICULES MÉTALLIQUES DANS DES FLUIDES

(30) Priorität: 08.01.2018 DE 102018000079
(43) Veröffentlichungstag der Anmeldung: 23.09.2020
(73) Patentinhaber: Hydac Electronic GmbH, 66128 Saarbrücken (DE)
(72) Erfinder: FUCHS, Patrik, 66459 Kirkel (DE); JACOBUS, Heinz, 66125 Dudweiler (DE)
(74) Vertreter: Bartels und Partner, Patentanwälte
(86) Internationale Anmeldenummer: PCT/EP2018/086569
(87) Internationale Veröffentlichungsnummer: WO 2019/134857

(56) Entgegenhaltungen:
- DE-U1- 20 320 334
- JP-A- 2010 014 518
- US-A- 5 782 141

## Beschreibung

Die Erfindung betrifft eine Sensorvorrichtung mit den Merkmalen im Oberbegriff von Patentanspruch 1.

Sensorvorrichtungen sind Stand der Technik, siehe EP 0 893 683 B1. Sensorvorrichtungen dieser Art kommen insbesondere bei Fluidsystemen zum Einsatz, bei denen Fluide, wie Hydrauliköle oder Schmieröle, entsprechende Systemkomponenten durchströmen, beispielsweise Hydropumpen und Hydromotoren oder Getriebegehäuse. Diese Systemkomponenten unterliegen dabei einem Verschleiß, wodurch das Fluid mit Metallpartikeln verunreinigt wird. Diese können sich in Ventilen, in durch das Fluid geschmierte Lager oder anderen Bauteilen festsetzen und dort Schäden verursachen. Die bekannten Sensorvorrichtungen ermöglichen das Erfassen der Verunreinigungen oder eines Verunreinigungsgrades der Fluide mit Metallpartikeln, indem der elektrische Widerstand zwischen zwei einander benachbarten Elektroden ermittelt wird, der vom Vorhandensein von zwischen den Elektroden befindlichen Metallpartikeln abhängig ist.

Im Stand der Technik ist hierbei, wie bei der in EP 0 893 683 B1 beschriebenen Lösung, ein Magnetfeld vorgesehen, um ferromagnetische Partikel an der Sensoreinrichtung anzusammeln. Mittels eines aufgrund des ermittelten Widerstandes generierten elektrischen Signals lässt sich das Fluidsystem abschalten, sobald der Verunreinigungsgrad einen vorgegebenen Schwellenwert überschritten hat, oder ein Alarmsignal erzeugen.

Systembedingt fallen beim Betrieb derartiger Sensorvorrichtungen Wartungsarbeiten an. Insbesondere muss nach einer zwischen den Elektroden des Sensors angehäuften Ansammlung von Metallpartikeln ein Reinigungsvorgang oder ein Austausch erfolgen, wozu die Sensoreinrichtung aus der den Fluidbereich vom Sensor trennenden Wand herausgenommen werden muss, also beispielsweise aus einer Rohrwand, Behälterwand oder Getriebegehäusewand. Da es normalerweise nicht praktikabel ist, den Fluidbereich für den jeweiligen Wartungsvorgang zu entleeren, gestaltet sich die Herausnahme der Sensoreinrichtung aus der Trennwand äußerst problematisch und birgt die Gefahr der Umweltgefährdung durch Auslaufen von Fluid.

Durch US 5 782 141 A ist eine Sensorvorrichtung mit einer Schließeinrichtung und einer Sensoreinrichtung zum Erfassen einer Verunreinigung in Form von Metallpartikeln in einem Fluid, das zumindest teilweise von einer Trennwand umgeben ist, die ein Leitungssystem oder ein vom Fluid durchströmtes Gehäuse begrenzt, wobei die Schließeinrichtung eine Aufnahmeöffnung aufweist, die ein zumindest teilweises Durchgreifen der Trennwand durch die in Funktionsstellung befindliche Sensoreinrichtung mit ihrem die Metallpartikel detektierenden Sensor ermöglicht, wobei die Schließeinrichtung auch dafür eingerichtet ist, die Sensoreinrichtung, insbesondere zu Wartungs- oder Austauschzwecken, in eine Außer-Funktionsstellung aus der Trennwand zu entfernen, wobei die Sensoreinrichtung derart mit der Schließeinrichtung zusammenwirkt, dass mit Entfernen der Sensoreinrichtung in ihre Außer-Funktionsstellung die Schließeinrichtung die Aufnahmeöffnung verschließt und in umgekehrter Richtung bei Einbringen der Sensoreinrichtung in ihre Funktionsstellung diese Aufnahmeöffnung freigibt, wobei die Schließeinrichtung unter Bildung einer Art Rückschlagventil einen federbelasteten Schließteller aufweist, der jeweils unter der Einwirkung der Sensoreinrichtung entgegen der Wirkung seiner Schließfeder in eine die Aufnahmeöffnung freigebende Öffnungsstellung oder unter der Wirkung der Schließfeder in seine die Aufnahmeöffnung schließende Sperrstellung bringbar ist, wobei die Schließfeder der Schließeinrichtung eine Druckfeder ist, die sich mit ihrem einen Ende an einem Aufnahmegehäuse und/oder Anlageteilen abstützt und mit ihrem anderen Ende an einem Gegenhalteteil angreift, das einstückiger Bestandteil der Schließeinrichtung ist oder mit der Schließeinrichtung verbindbar ist, und wobei der Schließteller und das Gegenhalteteil über ein stegartiges Verbindungsstück definiert auf einem Abstand zueinander gehalten sind.

Eine weitere Vorrichtung geht aus der DE 203 20 334 U1 hervor.

Im Hinblick auf diese Problematik stellt sich die Erfindung die Aufgabe, eine gattungsgemäße Sensorvorrichtung der zur Verfügung zu stellen, die sich durch ein verbessertes Betriebsverhalten auszeichnet. Erfindungsgemäß ist diese Aufgabe durch eine Sensorvorrichtung gelöst, die die Merkmale des Patentanspruchs 1 in seiner Gesamtheit aufweist.

Gemäß dem kennzeichnenden Teil des Anspruchs 1 zeichnet sich die Erfindung dadurch aus, dass das Verbindungsstück in Richtung auf das Gegenhalteteil Fluiddurchgänge freilässt, die mit einem weiteren Fluiddurchgang des Gegenhalteteils eine fluidführende Verbindung über die mittels der Schließeinrichtung freigegebene Aufnahmeöffnung zum Sensor der Sensoreinrichtung ermöglichen.

Entlang des Verbindungsstücks steht dadurch ein Einbauraum für die Druckfeder zur Verfügung, während gleichzeitig über die am Verbindungsstück und am Gegenhalteteil gebildeten Fluiddurchgänge die fluidführende Verbindung zum Sensor gebildet ist.

Es ist ferner vorgehen, dass die Sensoreinrichtung derart mit einer Schließeinrichtung zusammenwirkt, dass mit Entfernen der Sensoreinrichtung in ihre Außer-Funktionsstellung die Schließeinrichtung die Aufnahmeöffnung verschließt und in umgekehrter Richtung bei Einbringen der Sensoreinrichtung in ihre Funktionsstellung diese Aufnahmeöffnung freigibt. Dadurch, dass dergestalt der Vorgang des Entfernens der Sensoreinrichtung aus der Trennwand zu einem mittels der Schließeinrichtung bewirkten Verschluss der Aufnahmeöffnung führt und das Problem des Fluidaustritts dadurch aus der Welt geschaffen ist, gestalten sich Wartungs- oder Austauscharbeiten einfach und sicher, ohne dass die Gefahr einer Umweltbelastung besteht.

Die Schließeinrichtung weist unter Bildung einer Art Rückschlagventil einen federbelasteten Schließteller auf, der jeweils unter der Einwirkung der Sensoreinrichtung entgegen der Wirkung seiner Schließfeder in eine die Aufnahmeöffnung freigebende Öffnungsstellung oder unter der Wirkung der Schließfeder in seine die Aufnahmeöffnung schließende Sperrstellung bringbar ist. Dadurch ist eine beim Entfernen der Sensoreinrichtung selbsttätig schließende Schließeinrichtung in besonders einfacher Bauweise realisierbar.

Bei besonders vorteilhaften Ausführungsbeispielen nimmt das Aufnahmegehäuse permanent die Schließeinrichtung auf und ist in dieses die Sensoreinrichtung ein- und ausbringbar, insbesondere ein- und ausschraubbar . Die Schließeinrichtung bildet mit ihrem Aufnahmegehäuse dadurch einen Adapter, über den eine Sensorvorrichtung an einer gewählten Stelle der von dem zu überwachenden Fluid kontaktierten Trennwand anbringbar ist.

Der Schließteller der Schließeinrichtung kann außenumfangsseitig einen Dichtring aufweisen, der in seiner Schließstellung in dichtende Anlage mit dem Aufnahmegehäuse oder mit dem Aufnahmegehäuse zuordenbaren Anlageteilen ist. Unter Einfluss der Schließfeder ist dadurch ein sicherer, fluiddichter Verschluss der Aufnahmeöffnung bei entfernter Sensoreinrichtung gewährleistet.

Bei einem vorteilhaften Ausführungsbeispiel weist das Gegenhalteteil ein mit dem Verbindungsstück des Schließtellers verbindbares Schalenteil, das, zur Aufnahmeöffnung offen, eine Aufnahme für den zugewandten Bereich der Druckfeder bildet, sowie vom Schalenteil in Richtung auf die Sensoreinrichtung vorspringende Fußteile auf, zwischen denen zwischen den vom Schalenteil zur Sensoreinrichtung führende Fluiddurchgänge gebildet sind.

Für die Abstützung des äußeren Endes der Druckfeder kann am äußeren Ende des Aufnahmegehäuses das ringförmige Anlageteil angebracht sein, das die Aufnahmeöffnung begrenzt und die Dichtfläche für die dichtende Anlage des Dichtringes am Schließteller der Schließeinrichtung bildet.

Bei Ausbildung des Gegenhalteteils mit einem als gesondertes Bauteil herstellbaren Schalenteil kann für dessen Verbindung mit dem Verbindungsstück des Schließtellers eine Schnappverbindung vorgesehen sein, die einen einfachen Montagevorgang durch Verclipsen mit dem Verbindungsstück ermöglicht. Anstelle der Schnappverbindung kann auch ein üblicher Bajonettverschluss treten.

Nachstehend ist die Erfindung anhand von in der Zeichnung dargestellten Ausführungsbeispielen im Einzelnen erläutert. Es zeigen:
- Fig. 1: einen gegenüber einer praktischen Ausführungsform etwa um den Faktor 6 vergrößert gezeichneten Längsschnitt eines Ausführungsbeispiels der erfindungsgemäßen Vorrichtung, wobei der Betriebszustand der Funktionsstellung entspricht, bei der die Sensoreinrichtung in eine nur teilweise angedeutete Trennwand eingebracht ist;
- Fig. 2: einen der Fig. 1 entsprechenden Längsschnitt, wobei der Betriebszustand bei der Außer-Funktionsstellung dargestellt ist;
- Fig. 3: eine stärker vergrößert dargestellte perspektivische Schrägansicht des einen Schließteller und ein Gegenhalteteil bildenden Bauteils des Ausführungsbeispiels;
- Fig. 4: einen der Fig. 1 entsprechenden Längsschnitt eines zweiten Ausführungsbeispiels, wobei der Betriebszustand bei der Funktionsstellung gezeigt ist, und
- Fig. 5: einen der Fig. 1 entsprechenden Längsschnitt des zweiten Ausführungsbeispiels, wobei der Betriebszustand der Außer-Funktionsstellung dargestellt ist.

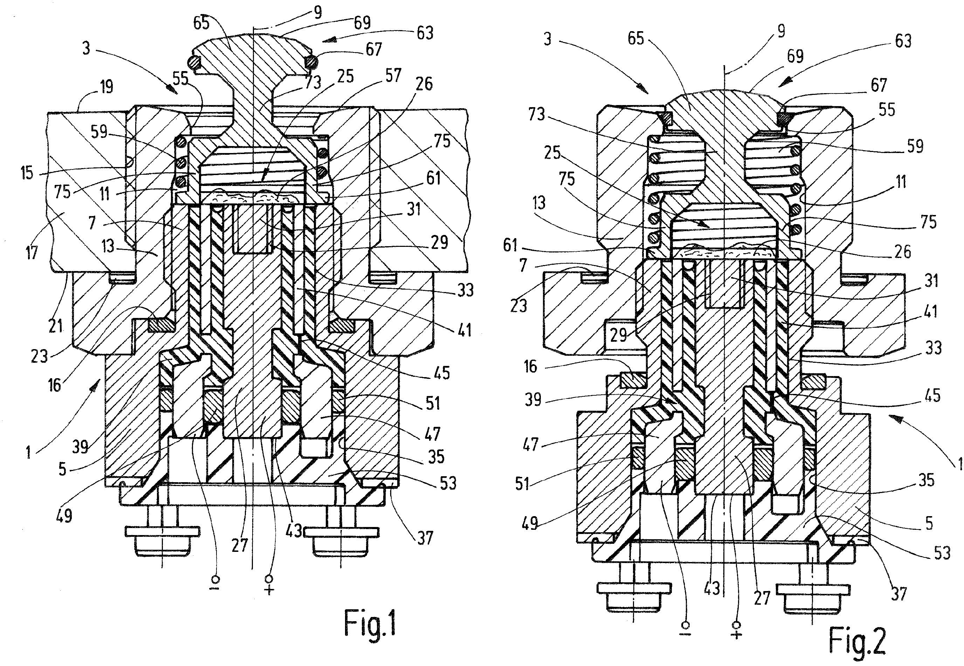

Die Fig. 1 zeigt ein Ausführungsbeispiel der Sensorvorrichtung im Betriebszustand der Funktionsstellung, wobei die Sensoreinrichtung 1 in die zugehörige Schließeinrichtung 3 eingebracht ist. Dabei ist das metallische Sensorgehäuse 5 der Sensoreinrichtung 1 mit einem Einschraubteil 7, das zur Längsachse 9 der Vorrichtung koaxial ist, in den Gewindeabschnitt einer zentralen Bohrung 11 in einem Aufnahmegehäuse 13 der Sensoreinrichtung 1 eingeschraubt. Ein Dichtring 16, der sich an einer Stufe am Ende des Einschraubteils 7 befindet, bildet hierbei die Abdichtung zwischen Sensorgehäuse 5 und Aufnahmegehäuse 13 der Schließeinrichtung 3. Das Aufnahmegehäuse 13 der Schließeinrichtung 3 ist, wie lediglich in Fig. 1 angedeutet ist, in eine Gewindebohrung 15 in einer Trennwand 17 eingeschraubt, an deren Innenseite 19 sich das auf Verunreinigungen zu überwachende Fluid befindet, wobei beispielsweise die Trennwand 17 ein Leitungssystem oder ein vom Fluid durchströmtes Gehäuse begrenzt. Das eingeschraubte Aufnahmegehäuse 13 ist gegenüber der Außenseite 21 der Trennwand 17 mittels eines Dichtringes 23 abgedichtet.

Der Sensor 25 der Sensoreinrichtung 3 weist innerhalb des Sensorgehäuses 5 einen zur Längsachse 9 koaxialen, metallischen Magnetträger 27 auf, der eine erste Elektrode des Sensors 25 bildet und in dessen am Ende des Einschraubteils 7 freiliegender Stirnseite ein in einer Aufnahme 29 des Magnetträger 27 aufgenommener Permanentmagnet 31 sitzt. Das Sensorgehäuse 5 weist einen inneren Durchgang auf, der, vom freien Ende des Einschraubteils 7 ausgehend, einen ersten, zur Achse 9 koaxialen Abschnitt 33 und einen sich daran anschließenden zweiten Abschnitt 35 aufweist, der sich bis zum offenen Ende 37 des Sensorgehäuses 5 erstreckt. In diesem Durchgang befindet sich ein Isolierkörper 39 in Form eines spritzgegossenen Kunststoffteils, beispielsweise aus PA6, mit dem der eine erste Sensor-Elektrode bildende Magnetträger 27, sowie ein nichtmagnetischer Metallzylinder 41 umspritzt sind, der eine zweite Sensor-Elektrode bildet.

Dieser Metallzylinder 41 ist in das Kunststoffmaterial des Isolierkörpers 39, zur Achse 9 koaxial, derart eingebettet, dass er an der freien Stirnseite des Einschraubteils 7 freiliegt und das ebenfalls freiliegende Ende des die erste Elektrode bildenden Magnetträgers 27 in geringem radialen Abstand umringt. Das von der den Permanentmagneten 31 aufweisenden Stirnseite entgegengesetzte untere Ende 43 des vom Sensorgehäuse 5 isolierten Magnetträgers 27 ist mittels einer nicht dargestellten Kontaktfeder mit einer elektrischen Potentialquelle verbunden, beispielsweise dem Pluspol einer Spannungsquelle. An das zu der oberen Stirnseite entgegengesetzte untere Ende des die zweite Elektrode bildenden Metallzylinders 41 schließt sich eine Bohrung 45 an, die als Durchgang für die Kunststoffmasse dient. Ein metallischer Haltering 47 bildet zusammen mit Dichtringen 49 und 51 den dichten Verschluss am unteren Abschnitt 35 des Durchgangs des Sensorgehäuses 5, wobei die Dichtringe 49 und 51 sowie der Ringkörper 47 durch einen Haltekörper 53 aus isolierendem Werkstoff, beispielsweise einem duroplastischen Kunststoff, festgelegt sind. Wie es auch beim die erste Elektrode bildenden Magnetträger 27 der Fall ist, ist auch der Ringkörper 47 über eine nicht gezeigte Kontaktfedereinrichtung mit einem Pol einer Spannungsquelle verbunden, beispielsweise mit dem Minuspol, wie es in Fig. 1 symbolisch angedeutet ist.

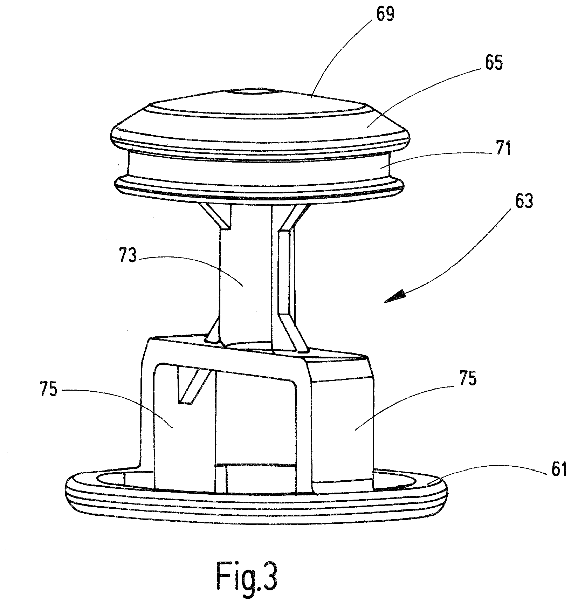

Die Bohrung 11 im Aufnahmegehäuse 13 der Schließeinrichtung 3 bildet mit ihrem zur Innenseite 19 der Trennwand 17 offenen Ende die Aufnahmeöffnung 55 für den Eintritt des zu überwachenden Fluids. Im an die Aufnahmeöffnung 55 angrenzenden Endbereich ist die Bohrung 11 nach innen eingezogen, so dass ein den Öffnungsdurchmesser verkleinernder Endrand gebildet ist, der mit einer radial nach innen vorspringenden Schulterfläche 57 eine Anlagefläche für ein Ende einer Druckfeder 59 bildet. Diese stützt sich mit ihrem anderen Ende an einem Gegenhalteteil 61 ab, das sich an einem Schließkörper 63 der Schließeinrichtung 3 befindet, der in Fig. 3 gesondert und vergrößert dargestellt ist und der zur Bildung eines Rückschlagventils in der Bohrung 11 des Aufnahmegehäuses 13 axial bewegbar angeordnet ist. Wie die Fig. 1 und 2 zeigen, ist der Schließkörper 63 durch die Druckfeder 59 mit seinem Gegenhalteteil 61 in Anlage an der Stirnfläche am Ende des Einschraubteils 7 des Sensorgehäuses 5 gehalten. Bei dem in Fig. 1 gezeigten Betriebszustand der Vorrichtung, bei dem das Sensorgehäuse 5 in das Aufnahmegehäuse 13 voll eingeschraubt ist, ist der Schließkörper 63 gegen die wirkende Federkraft in eine Öffnungsstellung bewegt, bei der ein Schließteller 65 des Schließkörpers 63 von der Aufnahmeöffnung 55 abgehoben ist.

Die Fig. 2 zeigt einen Betriebszustand, bei dem für die Entnahme des Sensorgehäuses 5 dieses bereits teilweise aus dem Aufnahmegehäuse 13 herausgeschraubt ist. Der Schließkörper 63 hat sich dadurch unter Wirkung der Druckfeder 59 mitbewegt, bis der Schließteller 65 mit seinem am Außenumfang befindlichen Dichtring 67 in der Schließstellung des Ventils in dichtende Anlage mit dem Rand der Aufnahmeöffnung 55 kommt. Wie am deutlichsten die Fig. 3 zeigt, weist der Schließteller 65 unterhalb seiner leicht konvex gewölbten Oberseite 69 außenumfangsseitig eine Ringnut 71 als Sitz des Dichtringes 67 auf, der in Fig. 3 weggelassen ist. Von der Unterseite des Schließtellers 65 erstreckt sich ein Verbindungsstück 73 als säulenartiger Träger des Schließtellers 65, wobei an das vom Schließteller 65 entfernte Ende des Verbindungsstücks 73 Stege 75 angeformt sind, die wiederum den Träger für das Gegenhalteteil 61 bilden. Letzteres hat die Form eines Kreisringes, dessen Außendurchmesser etwas geringer ist als der Innendurchmesser der Bohrung 11 des Aufnahmegehäuses 13, so dass eine Führung für die Verschiebebewegung des Schließkörpers 63 gebildet ist. Die Freiräume um das Verbindungsstück 73 und die Stege 75 bilden bei geöffneter

Schließeinrichtung 3 die Fluiddurchgänge, die über die Aufnahmeöffnung 55 zum Sensor 25 führen.

Die Fig. 1 zeigt den Betriebszustand der Funktionsstellung mit geöffneter Schließeinrichtung 3. Im Laufe des Betriebs der Vorrichtung führt das vom Permanentmagneten 31 am Sensor 25 erzeugte Magnetfeld bei Vorhandensein von ferromagnetischen Verschmutzungspartikeln des über die Aufnahmeöffnung 55 eintretenden Fluids zu einer Partikelansammlung an der Sensorstirnseite, wie es in den Figuren angedeutet und mit 26 bezeichnet ist. Durch diese Ansammlung kommt es, je nach mehr oder weniger großem Ausmaß der Ansammlung 26 und damit zusammenhängender Höhe des Verschmutzungsgrades des Fluids, zu einer elektrischen Verbindung zwischen dem Magnetträger 27 als erster Elektrode und dem umringenden Metallzylinder 41 als zweiter Elektrode, wobei die Abnahme des elektrischen Widerstands zwischen diesen Elektroden vom Ausmaß des Vorhandenseins metallischer Partikel abhängig ist. Wenn ein einen gegebenen Schwellenwert übersteigender Verschmutzungsgrad erkannt ist und entsprechende Maßnahmen zu treffen sind, wobei die Partikelansammlung 26 vom Sensor 25 zu entfernen ist, wird die Vorrichtung durch Herausschrauben des Sensorgehäuses 5 aus dem Aufnahmegehäuse 13 in die Außer-Funktionsstellung gebracht. Fig. 2 zeigt den Beginn des Herausschraubens, wobei der Schließkörper 73 unter Einfluss der Druckfeder 59 der Axialbewegung des Sensorgehäuses 5 folgt und der Schließteller 65 in die die Aufnahmeöffnung 55 schließende Sperrstellung gelangt, s. Fig. 2, so dass Wartungs- oder Austauschmaßnahmen, wie das Entfernen der Ansammlung 26, ohne Fluidaustritt erfolgen können.

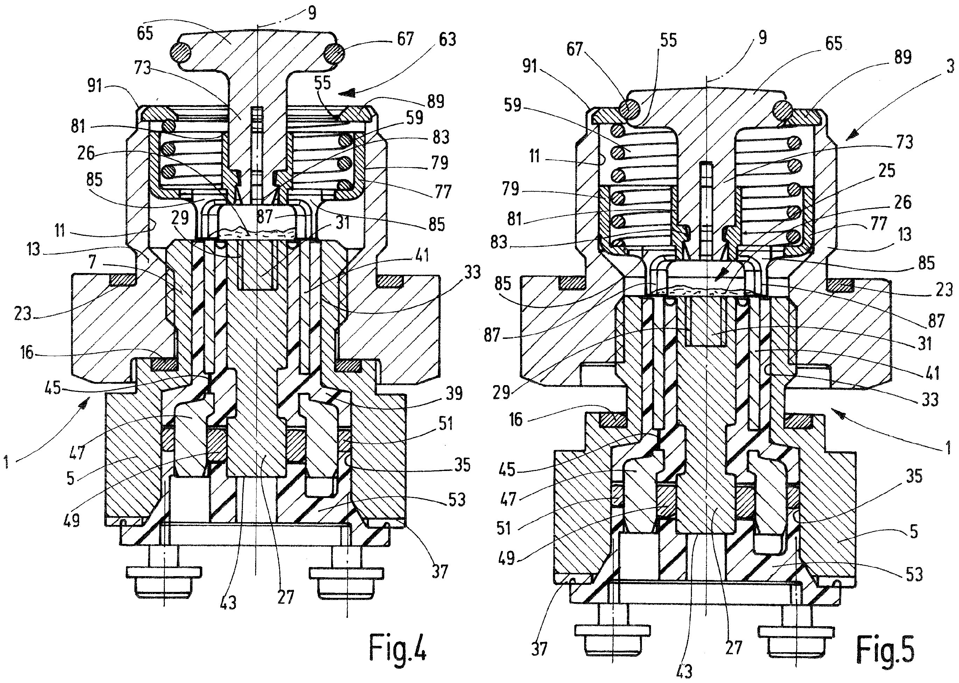

Das in Fig. 4 und 5 dargestellte zweite Ausführungsbeispiel unterscheidet sich vom ersten Ausführungsbeispiel hauptsächlich durch eine abgewandelte Bauweise der Schließeinrichtung 3, während das Sensorgehäuse 5 mit seinen zugehörigen Bauelementen unverändert dem ersten Beispiel entspricht. Anders als beim ersten Ausführungsbeispiel, wo der Schließkörper 63 als einteiliges spritzgeformtes Bauteil aus einem Kunststoffwerkstoff, wie PA6, beispielsweise mit Glasfaserverstärkung, oder aus einem metallischen Werkstoff gebildet ist, ist der Schließkörper 63 beim zweiten Ausführungsbeispiel aus solchen Werkstoffen zweiteilig ausgebildet. Anstelle des beim ersten Ausführungsbeispiel an den Stegen 75 des Verbindungsstücks 73 unmittelbar angeformten Gegenhalteteils 61 ist beim zweiten Ausführungsbeispiel das Gegenhalteteil durch ein Schalenteil 77 gebildet, das mittels einer Schnappverbindung an dem Verbindungsstück 73 des Schließkörpers 63 angebracht ist, das sich in Form eines zylindrischen Zapfens vom Schließteller 65 weg erstreckt. Das Schalenteil 77 hat die Form einer in Richtung auf den Schließteller 65 offenen runden Schale, die mit ihrer Seitenwand 79 in der Bohrung 11 des Aufnahmegehäuses 13 axial beweglich geführt ist. Vom Zentralbereich des Schalenbodens erstreckt sich ein Hülsenteil 81 weg, das den unteren Längenabschnitt des Verbindungsstücks 73 umgibt und mit nach innen vorspringenden Noppen 83, die mit Kerben im Verbindungsstück 73 verclipsbar sind, die Schnappverbindung bilden. Neben Durchgängen 85 im Boden des Schalenteils 77 erstrecken sich Fußteile 87 nach unten, die unter der Wirkung der Druckfeder 59, die sich auf dem Boden des Schalenteils 77 als dem Gegenhalteteil abstützt, in Anlage an der Stirnfläche des Sensors 25 am Ende des Einschraubteils 7 des Sensorgehäuses 5 gehalten sind. Anders als beim ersten Ausführungsbeispiel, wo das Anlageteil für das obere Ende der Druckfeder 59 durch eine Schulterfläche 57 am Aufnahmegehäuse 13 gebildet ist, ist beim vorliegenden zweiten Ausführungsbeispiel ein gesondertes Anlageteil in Form eines Kreisringes 89 vorgesehen, der die Aufnahmeöffnung 55 begrenzt und an dem freien Endrand 91 des Aufnahmegehäuses 13 durch Verstemmen festgelegt ist. Der Kreisring 89 bildet die Dichtfläche, die bei der Schließstellung des gebildeten Rückschlagventils mit dem Dichtring 67 am Schließteller 65 zusammenwirkt.

Die Funktionsweise des zweiten Ausführungsbeispiels entspricht derjenigen des ersten Ausführungsbeispiels. Wie beim ersten Beispiel, wo der Fluidweg von der geöffneten Aufnahmeöffnung 55 zur Stirnseite des Sensors 25 über die zwischen den Stegen 75 am Verbindungsstück 73 des Schließkörpers 63 gebildeten Freiräume erfolgt, ist auch beim zweiten Ausführungsbeispiel ein Fluidweg aus der Innenseite des Schalenteils 77 über die im Schalenboden gebildeten Durchgänge 85 zwischen den Fußteilen 87 gebildet. Für den Einsatz bei Fluiden mit einem entsprechenden Druckniveau, beispielsweise im Bereich von 10 bis 30 bar, vorzugsweise von 16 bar, ist das aus den miteinander verschraubten Gehäuseteilen, Sensorgehäuse 5 und Aufnahmegehäuse 13, gebildete Gesamtgehäuse druckfest und mittels der Dichtelemente 16, 23, 49 und 51 druckdicht ausbildbar, wobei als Gehäusewerkstoff Edelstahl oder verzinkter Stahl vorteilhaft in Frage kommen.

## Patentansprüche

1. Sensorvorrichtung mit einer Schließeinrichtung (3) und einer Sensoreinrichtung (1) zum Erfassen einer Verunreinigung in Form von Metallpartikeln in einem Fluid, das zumindest teilweise von einer Trennwand (17) umgeben ist, die ein Leitungssystem oder ein vom Fluid durchströmtes Gehäuse begrenzt,
wobei die Schließeinrichtung (3) eine Aufnahmeöffnung (55) aufweist, die ein zumindest teilweises Durchgreifen der Trennwand (17) durch die in Funktionsstellung befindliche Sensoreinrichtung (1) mit ihrem die Metallpartikel detektierenden Sensor (25) ermöglicht,
wobei die Schließeinrichtung (3) auch dafür eingerichtet ist, die Sensoreinrichtung (1), insbesondere zu Wartungs- oder Austauschzwecken, in eine Außer-Funktionsstellung aus der Trennwand (17) zu entfernen,
wobei die Sensoreinrichtung (1) derart mit der Schließeinrichtung (3) zusammenwirkt, dass mit Entfernen der Sensoreinrichtung (1) in ihre Außer-Funktionsstellung die Schließeinrichtung (3) die Aufnahmeöffnung (55) verschließt und in umgekehrter Richtung bei Einbringen der Sensoreinrichtung (1) in ihre Funktionsstellung diese Aufnahmeöffnung (55) freigibt,
wobei die Schließeinrichtung (3) unter Bildung einer Art Rückschlagventil einen federbelasteten Schließteller (65) aufweist, der jeweils unter der Einwirkung der Sensoreinrichtung (1) entgegen der Wirkung seiner Schließfeder (59) in eine die Aufnahmeöffnung (55) freigebende Öffnungsstellung oder unter der Wirkung der Schließfeder (59) in seine die Aufnahmeöffnung (55) schließende Sperrstellung bringbar ist,
wobei die Schließfeder der Schließeinrichtung (3) eine Druckfeder (59) ist, die sich mit ihrem einen Ende an einem Aufnahmegehäuse (13) und/oder Anlageteilen (89) abstützt und mit ihrem anderen Ende an einem Gegenhalteteil (61; 77) angreift, das einstückiger Bestandteil der Schließeinrichtung (3) ist oder mit der Schließeinrichtung (3) verbindbar ist, und
wobei der Schließteller (65) und das Gegenhalteteil (61; 77) über ein stegartiges Verbindungsstück (73) definiert auf einem Abstand zueinander gehalten sind,
**dadurch gekennzeichnet,**
**dass** das Verbindungsstück (73) in Richtung auf das Gegenhalteteil (61; 77) Fluiddurchgänge freilässt, die mit einem weiteren Fluiddurchgang (75; 85) des Gegenhalteteils (61; 77) eine fluidführende Verbindung über die mittels der Schließeinrichtung (3) freigegebene Aufnahmeöffnung (55) zum Sensor (25) der Sensoreinrichtung (1) ermöglichen.

2. Sensorvorrichtung nach Anspruch 1, **dadurch gekennzeichnet, dass** das Aufnahmegehäuse (13) permanent die Schließeinrichtung (3) aufnimmt und in dieses die Sensoreinrichtung (1) ein- und ausbringbar, insbesondere ein- und ausschraubbar, ist.

3. Sensorvorrichtung nach Anspruch 1 oder 2, **dadurch gekennzeichnet, dass** der Schließteller (65) der Schließeinrichtung (3) außenumfangsseitig einen Dichtring (67) aufweist, der in seiner Schließstellung in dichtende Anlage mit dem Aufnahmegehäuse (13) oder mit dem Aufnahmegehäuse (13) zuordenbaren Anlageteilen (89) ist.

4. Sensorvorrichtung nach einem der vorstehenden Ansprüche, **dadurch gekennzeichnet, dass** das Gegenhalteteil ein mit dem Verbindungsstück (73) des Schließtellers (65) verbindbares Schalenteil (77), das, zur Aufnahmeöffnung (55) offen, eine Aufnahme für den zugewandten Bereich der Druckfeder (59) bildet, sowie vom Schalenteil (77) in Richtung auf die Sensoreinrichtung (1) vorspringende Fußteile (87) aufweist, zwischen denen vom Schalenteil (77) zur Sensoreinrichtung (1) führende Fluiddurchgänge (85) gebildet sind.

5. Sensorvorrichtung nach Anspruch 4, **dadurch gekennzeichnet, dass** am äußeren Ende des Aufnahmegehäuses (13) für die Abstützung des äußeren Endes der Druckfeder (59) das ringförmige Anlageteil (89) angebracht ist, das die Aufnahmeöffnung (55) begrenzt und die Dichtfläche für die dichtende Anlage des Dichtringes (67) am Schließteller (65) der Schließeinrichtung (3) bildet.

6. Sensorvorrichtung nach Anspruch 4 oder 5, **dadurch gekennzeichnet, dass** das Schalenteil (77) mit dem Verbindungsstück (73) des Schließtellers (65) mittels einer Schnappverbindung (83) verclipst ist.

## Claims

1. Sensor device having a closing apparatus (3) and a sensor apparatus (1) for detecting contamination in the form of metal particles in a fluid, which is at least partially surrounded by a separating wall (17), which delimits a pipe system or a housing through which the fluid flows,
wherein the closing apparatus (3) comprises a receiving opening (55), which allows the sensor apparatus (1) located in functional position, with its sensor (25) detecting the metal particles, to engage at least partially through the separating wall (17),
wherein the closing apparatus (3) is also configured to remove the sensor apparatus (1) from the separating wall (17), in particular for maintenance or replacement purposes, into a non-functional position,
wherein the sensor apparatus (1) interacts with the closing apparatus (3) such that, when the sensor apparatus (1) is removed into its non-functional position, the closing apparatus (3) closes the receiving opening (55) and, in the reverse direction, when the sensor apparatus (1) is moved into its functional position, the closing apparatus releases this receiving opening (55),
wherein the closing apparatus (3), forming a kind of non-return valve, comprises a spring-loaded obturator disc (65), which can respectively be moved under the influence of the sensor apparatus (1) against the action of its closing spring (59) into an opening position releasing the receiving opening (55) or can be moved under the influence of the closing spring (59) into its blocking position closing the receiving opening (55),
wherein the closing spring of the closing apparatus (3) is a compression spring (59), which is supported with one of its ends on a receiving housing (13) and/or bearing parts (89) and with its other end engages on a brace part (61; 77), which is an integral component of the closing apparatus (3) or can be connected to the closing apparatus (3), and
wherein the obturator disc (65) and the brace part (61; 77) are held a defined distance away from one another by means of a web-like connection piece (73),
**characterised in that**
the connection piece (73) releases fluid passages in the direction of the brace part (61; 77), which, with a further fluid passage (75; 85) of the brace part (61; 77), permit a fluid-conveying connection via the receiving opening (55) released by means of the closing apparatus (3) to the sensor (25) of the sensor apparatus (1).

2. Sensor device according to claim 1, **characterised in that** the receiving housing (13) permanently receives the closing apparatus (3) and the sensor apparatus (1) can be moved into and out of said housing, particularly screwed into and out of said housing.

3. Sensor device according to either claim 1 or claim 2, **characterised in that** the obturator disc (65) of the closing apparatus (3) comprises a sealing ring (67) on its outer circumferential side, which, in its closing position, is in sealing contact with the receiving housing (13) or with bearing parts (89) that can be assigned to the receiving housing (13).

4. Sensor device according to any of the preceding claims, **characterised in that** the brace part comprises a shell part (77) that can be connected to the connection piece (73) of the obturator disc (65), said shell part being open to the receiving opening (55) and forming a receptacle for the facing region of the compression spring (59), and foot parts (87) protruding from the shell part (77) in the direction of the sensor apparatus (1), between which fluid passages (85) leading from the shell part (77) to the sensor apparatus (1) are formed.

5. Sensor device according to claim 4, **characterised in that** the annular bearing part (89) is attached to the outer end of the receiving housing (13) to support the outer end of the compression spring (59), said bearing part delimiting the receiving opening (55) and forming the sealing face for sealing contact of the sealing ring (67) on the obturator disc (65) of the closing apparatus (3).

6. Sensor device according to either claim 4 or claim 5, **characterised in that** the shell part (77) is clipped to the connection piece (73) of the obturator disc (65) by means of a snap-fit connection (83).

## Revendications

1. Appareil de détection comprenant un dispositif (3) de fermeture et un dispositif (1) de détection pour la détection d'une impureté sous la forme de particules métalliques dans un fluide, lequel est entouré au moins en partie d'une cloison (17), qui délimite un système de conduit ou une enveloppe dans laquelle passe le fluide,
dans lequel le dispositif (3) de fermeture a une ouverture (55) de réception, qui rend possible une traversée au moins en partie de la cloison (17) par le dispositif (1) de détection se trouvant dans une position de fonctionnement avec son détecteur (25) détectant les particules métalliques,
dans lequel le dispositif (3) de fermeture est agencé également pour éloigner de la cloison (17), dans une position de fonctionnement à l'extérieur, le dispositif (1) de détection, en particulier à des fins d'entretien ou de remplacement,
dans lequel le dispositif (1) de détection coopère avec le dispositif (3) de fermeture, de manière à ce que, par l'éloignement du dispositif (1) de détection dans sa position de fonctionnement à l'extérieur, le dispositif (3) de fermeture ferme l'ouverture (55) de réception et en sens contraire, lorsque le dispositif (1) de détection est mis dans sa position de fonctionnement, dégage cette ouverture (55) de réception,
dans lequel le dispositif (3) de fermeture a, en formant une sorte de clapet anti-retour, une assiette (65) de fermeture soumise à l'action d'un ressort, qui, respectivement sous l'action du dispositif (1) de détection, peut, à l'encontre de l'effet de son ressort (59) de fermeture, être mise dans une position d'ouverture dégageant l'ouverture (55) de réception ou, sous l'effet du ressort (59) de fermeture, dans sa position d'arrêt fermant l'ouverture (55) de réception,
dans lequel le ressort de fermeture du dispositif (3) de fermeture est un ressort (59) de compression, qui s'appuie par l'une de ses extrémités sur une enveloppe (13) de réception et/ou des parties (89) d'appareil et par son autre extrémité attaque une partie (61 ; 77) de contre-support, qui est une partie constitutive d'une seule pièce du dispositif (3) de fermeture ou qui peut être reliée au dispositif (3) de fermeture, et
dans lequel l'assiette (65) de fermeture et la partie (61 ; 77) de contre-support sont maintenues à une distance l'une par rapport à l'autre définie par une pièce (73) de liaison de type à entretoise,
**caractérisé**
**en ce que** la pièce (73) de liaison dégage, dans la direction de la partie (61 ; 77) de contre-support, des passages pour du fluide, qui rendent possible, par un autre passage (75 ; 85) pour du fluide de la partie (61 ; 77) de contre-support, une liaison fluidique menant au détecteur (25) du dispositif (1) de détection, en passant par l'ouverture (55) de réception dégagée au moyen du dispositif (3) de fermeture.

2. Appareil de détection suivant la revendication 1, **caractérisé en ce que** l'enveloppe (13) de réception reçoit en permanence le dispositif (3) de fermeture et le dispositif (1) de détection peut y être mis et en être sorti, en particulier y être vissé et dévissé.

3. Appareil de détection suivant la revendication 1 ou 2, **caractérisé en ce que** l'assiette (65) de fermeture du dispositif (3) de fermeture a, du côté du pourtour extérieur, une bague (67) d'étanchéité, qui, dans sa position de fermeture, est en contact étanche avec l'enveloppe (13) de réception ou avec des parties (89) d'appareil pouvant être associées à l'enveloppe (13) de réception.

4. Appareil de détection suivant l'une des revendications précédentes, **caractérisé en ce que** la partie de contre-support forme, avec la partie (77) de coque, qui peut être reliée à la pièce (73) de liaison de l'assiette (65) de fermeture, un logement, ouvert vers l'ouverture (55), de réception de la partie tournée vers elle du ressort (59) de compression ainsi qu'a des parties (87) de pied, qui sont en saillie de la partie (77) de coque dans la direction du dispositif (1) de détection et entre lesquelles sont formées des passages (85) pour du fluide allant de la partie (77) de coque au dispositif (1) de détection.

5. Appareil de détection suivant la revendication 4, **caractérisé en ce que**, à l'extrémité extérieure de l'enveloppe (13) de réception est disposée, pour l'appui de l'extrémité extérieure du ressort (59) de compression, la partie (89) annulaire d'appareil, qui délimite l'ouverture (55) de réception et qui forme la surface d'étanchéité pour l'application avec étanchéité de la bague (67) d'étanchéité à l'assiette (65) de fermeture du dispositif (3) de fermeture.

6. Appareil de détection suivant la revendication 4 ou 5, **caractérisé en ce que** la partie (77) de coque est clipsée à la pièce (73) de liaison de l'assiette (65) de fermeture au moyen d'une liaison (83) à déclic.
